# EUROPEAN PATENT SPECIFICATION

(11) **EP 0 659 384 B1**
(45) Date of publication and mention of the grant of the patent: **20.02.2002**
(21) Application number: 94309563.8
(22) Date of filing: 20.12.1994
(51) Int. Cl.: A61B 5/0245

(54) **Pulse rate monitor**
Puls - Monitor
Moniteur du poulse

(30) Priority: 20.12.1993 JP 32015793; 06.12.1994 JP 30251194
(43) Date of publication of application: 28.06.1995
(73) Proprietor: SEIKO INSTRUMENTS INC., Chiba-shi, Chiba 261 (JP)
(72) Inventor: Odagiri, Hiroshi, c/o Seiko Instruments Inc., Chiba-shi, Chiba (JP); Sakumoto, Kazumi, c/o Seiko Instruments Inc., Chiba-shi, Chiba (JP); Tsubata, Keisuke, c/o Seiko Instruments Inc., Chiba-shi, Chiba (JP); Nosaka, Naokatsu, c/o Seiko Instruments Inc., Chiba-shi, Chiba (JP); Nakamura, Chiaki, c/o Seiko Instruments Inc., Chiba-shi, Chiba (JP); Hayakawa, Motomu, c/o Seiko Epson Corporation, Suwa-shi, Nagano (JP)
(74) Representative: Sturt, Clifford Mark

(56) References cited:
- WO-A-91/18550
- DE-A- 3 150 925
- DE-A- 4 001 574
- US-A- 4 911 427
- US-A- 5 025 791
- US-A- 5 243 992

## Description

The present invention relates to a pulse rate monitor mounted on a portion of a human or other biological body.

In many circumstances it is desirable to be provided with an indication of the pulse rate of a human or other biological body. For example, during exercise, such as running, a person may wish to monitor their pulse rate in order to obtain an indication of their physical fitness and/or the vigorousness of the excercise. However, the movement/actions of the body make it difficult to accurately determine the pulse rate.

DE 3150925A discloses an arrangement for pulse measurement, comprising a pulse wave sensor for detecting a pulse from a biological body and generating a signal indicative thereof; an interference movement sensor for sensing movement of the body and generating a signal indicative thereof; and a differential amplifier for subtracting the amplified movement signals from the amplified pulse signals.

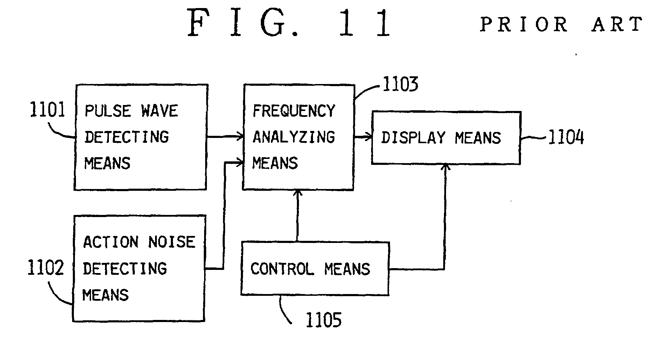

Figure 11 is a functional block diagram representing the construction of the conventional pulse wave detecting apparatus. The pulse wave detecting means 1101 detects a pulse wave signal, and outputs a detected pulse wave signal to a frequency analyzing means 1103. The action noise detecting means 1102 detects an action noise signal and outputs a detected action noise signal to the frequency analyzing means 1103. The display means 1104 displays the frequency-analyzed result made by the frequency analyzing means 1103. The control means 1105 controls the frequency analyzing means 1103 and the display means 1104.

The above-described pulse wave analyzing apparatus is disclosed in Japanese Laid-Open Patent No. 60-259239.

WO 91/18550 discloses a pulse responsive device comprising a light emitter and receiver for providing an electrical signal varying in accordance with blood flow pulsations and a movement transducer for providing an electrical signal representing body movement. The device cancels out the movement noise from the signal varying in accordance with blood flow pulsations.

However, the conventional pulse rate monitor merely displays both the frequency analyzed result of the pulse wave sensor and the frequency analyzed result of the action noise sensor on the output display unit. As a result, either the pulse wave frequency, or the pulse rate, must be determined by the user by observing the respective result representations on the output display unit. Also, the same signal processing system is used to determine the pulse wave frequency-analyzed and action noise frequency-analyzed results when the user is stationary and moving and thus, unnecessary parts of the signal processing unit are operated under the stationary condition.

It is an object of the present invention to provide a pulse rate monitor capable of displaying as an output a pulse rate representation even when the user is moving. Furthermore, another object of the present invention is to provide a pulse rate monitor capable of suppressing power consumption by reducing the amount by which signals are processed under a stationary condition, thereby shortening calculation time and saving power.

According to one aspect of the invention, there is provided a pulse rate monitor comprising:
a pulse wave sensor for detecting a pulse from a biological body and generating a signal indicative thereof;
a movement sensor for sensing movement of the biological body and generating a signal indicative thereof;
frequency analysing means for resolving the pulse wave indicative signal and movement indicative signal into frequency components (fmg, fsg); and
pulse wave component extracting means for operatively identifying the pulse rate of the biological body from the pulse wave frequency components and movement frequency components; characterised in that:
   said movement sensor comprises an action noise sensor for detecting an action noise corresponding to a biological body action, and for outputting a movement signal derived from said action noise; and
   said pulse wave component extracting means is adapted to determine the pulse rate in accordance with a said biological body action by extracting the typical pulse wave component from the pulse wave components and the movement signal components and further comprises pulse rate calculating means for calculating pulse rate per predetermined period of the biological body based on the typical pulse wave component extracted by said pulse wave component extracting means.

The pulse wave sensor and/or movement sensor may be mounted on or in a head, wrist, waist or leg band or the like. The sensors may be mounted on or in separate items or the same item. One or more of the sensors may be included in a watch wrist strap. In any event, the pulse wave sensor should be mounted on the body such that it can detect a pulse.

According to another aspect of the invention, there is provided an action pitch monitor comprising:
action noise detecting means having an action noise sensor for detecting an action noise corresponding to a biological body action, and for outputting an action noise signal;
action noise signal converting means for converting the action noise signal into a digital signal;
action noise signal storing means for storing the digital signal converted by said action noise signal converting means;
action noise signal calculating means for processing the digital signal stored in said action noise signal storing means to obtain the action noise components representing frequency and power spectrum of the action noise signal;
action noise component extracting means for extracting a typical action noise component from the action noise components; and
action pitch calculating means for calculating action pitch, per predetermined period, of the biological body based on the typical action noise components extracted by said action noise component extracting means.

A movement sensor/action noise detection means is provided for supplying signals to a processor to determine a movement characteristic/action pitch.

A pulse rate monitor may be arranged by employing pulse wave component extracting means for extracting pulse wave components from frequency analyzing results of a pulse wave sensor and an action noise sensor; and pulse rate calculating means for calculating a pulse rate per one minute based on the pulse wave components extracted from the pulse wave component extracting means. Further, the pulse rate monitor may be arranged to confirm whether or not the action noise signal exists by action noise detecting means; and for changing the calculation method by calculation changing means when no action noise signal exists during a predetermined period of time. As a result, the calculation amount can be reduced and the power consumption can be suppressed, so that the calculation time can be shortened and power can be saved.

For a better understanding of the invention, embodiments of the invention will now be described by way of example with reference to the accompanying drawings, in which:-
Figure 1 is a functional block diagram for representing an example of an arrangement of a prior art pulse rate monitor;
Figure 2 is a functional block diagram for representing an example of the arrangement of a pulse rate monitor;
Figure 3 is a functional block diagram for showing a pulse rate monitor according to an embodiment of the present invention;
Figure 4 is a flow chart for showing an operation concept of CPU in the pulse rate monitor according to the embodiment of the present invention;
Figure 5A shows an example of a waveform to be FFT-processed;
Figure 5B indicates a result of the FFT-processed waveform;
Figure 6A shows a result of the FFT-processed output signal of the pulse wave sensor;
Figure 6B indicates a result of the FFT-processed output signal of the action noise sensor;
Figure 6C represents such a pulse wave component obtained by subtracting the result of Figure 6B from the result of Figure 6A;
Figure 7 is a flow chart for indicating one example of a method for specifying the pulse wave component of the pulse rate monitor;
Figure 8 is a flow chart for indicating another example of a method for specifying the pulse wave component of the pulse rate monitor;
Figure 9 is a flow chart for indicating another example of a method for specifying the pulse wave component of the pulse rate monitor;
Figure 10 is a flow chart for indicating another example of a method for specifying the pulse wave component of the pulse rate monitor;
Figure 11 is a functional block diagram for indicating the arrangement of the conventional pulse rate monitor;
Figures 12A-12C are explanatory diagrams for indicating examples of outer appearance of the pulse rate monitor; Figure 12A shows a pulse rate monitor having the pulse wave sensor in a finger sack; Figure 12B indicates a pulse rate monitor having the action noise sensor assembled into a belt; and Figure 12C indicates a pulse rate monitor having the pulse wave sensor and the action noise sensor assembled into a watch strap;
Figure 13 represents a result of the FFT-processed output signal from the action noise sensor; and
Figure 14 indicates an example of a functional structural diagram of a motion pitch monitor.

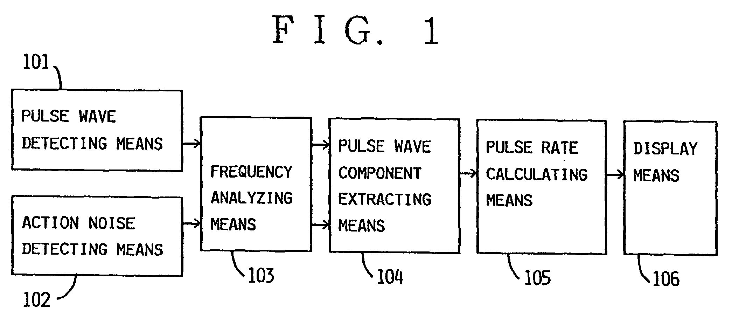

Figure 1 is a functional block diagram representing one example of a pulse rate monitor.

The pulse wave detecting means 101 detects a pulse wave from a human or animal body, for example, and outputs the detected signal to the frequency analyzing means 103. The action noise detecting means 102 detects movement of the body and outputs the detected signal to the frequency analyzing means 103. The frequency analyzing means 103 frequency-analyzes the output signals from the pulse wave detecting means 101 and the action noise detecting means 102. To perform the frequency analysis, for instance, the Fast Fourier Transform (FFT) is employed.

The pulse wave component extracting means 104 specifies a pulse wave component corresponding to a pulse rate frequency from the frequency-analyzed results output from the pulse wave detecting means 101 and the action noise detecting means 102, and outputs the specified pulse wave component to the pulse rate calculating means 105. The pulse rate calculating means 105 calculates a pulse rate per minute from the frequency component of the pulse rate specified by the pulse wave component extracting means 104. The display means 106 displays the pulse rate calculated by the pulse rate calculating means 105.

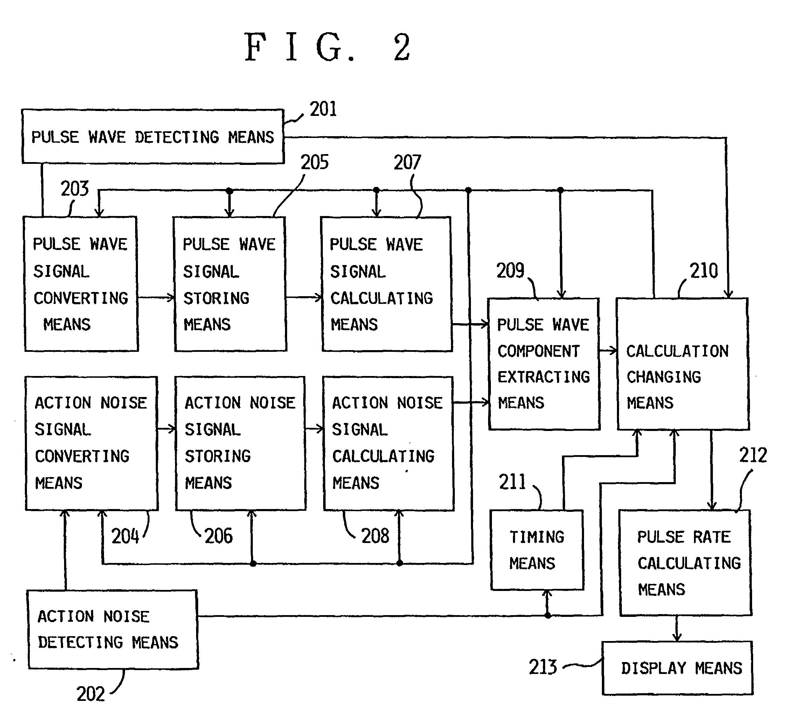

Figure 2 is a functional view for representing another example of the arrangement of a pulse rate monitor. Pulse wave signal converting means 203 converts an analog pulse wave voltage signal of a human body, detected by the pulse wave detecting means 201, into a digital signal. The converted digital signal is output into pulse wave signal storing means 205.

The action noise converting means 204 converts an analog voltage signal proportional to a magnitude of the action noise, detected by the action noise detecting means 202, into a digital signal. The converted digital signal is output into action noise signal storing means 206. The pulse wave signal storing means 205 stores therein the pulse wave signal converted into the digital signal. The action noise signal storing means 206 stores therein the action noise signal converted into the digital signal.

Pulse wave signal calculating means 207 sequentially reads out the pulse wave signals stored in the pulse wave signal storing means 205 and frequency-analyzes the read pulse wave signals, thereby outputting the frequency-analyzed result into pulse wave component extracting means 209. Action noise signal calculating means 208 sequentially reads out the action noise signals stored in the action noise signal storing means 206 and performs a frequency analysis of the read action noise signal, thereby outputting the frequency-analyzed result into the pulse wave component extracting means 209. The pulse wave component extracting means 209 extracts only the pulse wave components from the frequency-analyzed results by the pulse wave signal calculating means 207 and the action noise signal calculating means 208, thereby outputting these extracted pulse wave components to calculation changing means 210.

Timing means 211 counts a time period during which there is no action noise signal that has been shaped into rectangular waves by the action noise detecting means 202), and indicates that the counted time period exceeds a predetermined time period to the calculation changing means 210. Upon receipt of the no action noise signal issued from the timing means 211, the calculation changing means 210 outputs the pulse wave signal (which has been shaped into rectangular waves by the pulse wave detecting means 201) into pulse rate calculating means 212. At the same time, the calculation changing means 210 stops the operations of the pulse wave signal converting means 203, pulse wave signal storing means 205, pulse wave signal calculating means 207, action noise signal converting means 204, action noise signal storing means 206, action noise signal calculating means 208, and pulse wave component extracting means 209.

While the calculation changing means 210 outputs the rectangular shaped pulse wave signal to the pulse rate calculating means 212, when the action noise is detected by the action noise detecting means 202, the calculation changing means 210 immediately restarts the operations of the above-described pulse wave signal converting means 203, pulse wave signal storing means 205, pulse wave signal calculating means 207, action noise signal converting means 204, action noise signal storing means 206, action noise signal calculating means 208, and pulse wave component extracting means 209, and also changes into the output signal of the pulse wave component extracting means 209.

In the pulse rate calculating means 212, a pulse rate per minute is calculated from either the frequency component of the pulse wave extracted by the pulse wave component extracting means 209, or the periodic data of the pulse wave which has been shaped by the pulse wave detecting means 201, and the calculation result is output to the display means 213.

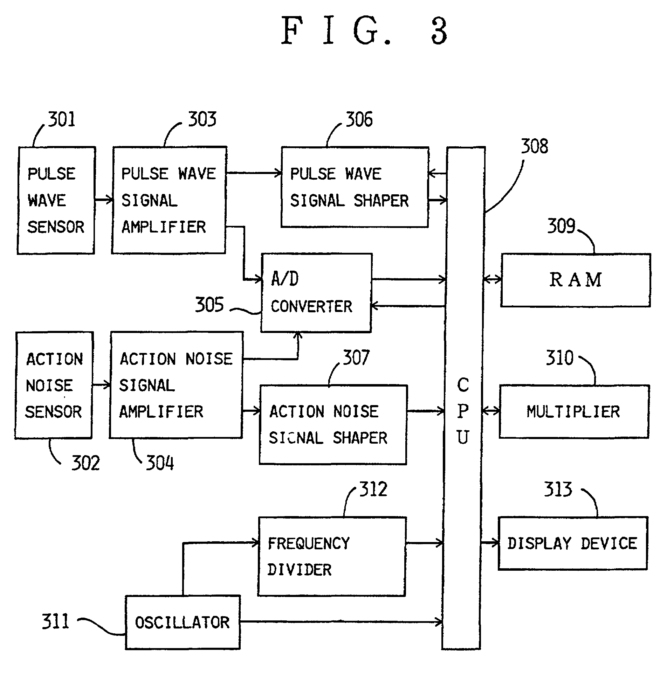

Figure 3 is a functional block diagram representing a pulse rate monitor according to a first embodiment of the present invention. A pulse wave sensor 301 senses a pulse wave from a biological body, and outputs the sensed pulse wave signal to a pulse wave signal amplifier 303. An action noise sensor 302 senses movement of the biological body and outputs the sensed action noise signal to an action noise signal amplifier 304.

As the pulse wave sensor, for instance, a piezoelectric microphone is employed. As the action noise sensor, an acceleration sensor is utilized, for example.

The pulse wave signal amplifier 303 amplifies the pulse wave signal and outputs the amplified pulse wave signal to an A/D converter 305 and a pulse wave shaper 306. The action noise signal amplifier 304 amplifies the action noise signal and outputs the amplified action noise signal to the AID converter 305 and an action noise signal shaper 307. The A/D converter 305 converts both the pulse wave signal and the action noise signal to output the A/D-converted pulse wave signal and action noise signal to a CPU 308. The pulse wave signal shaper 306 shapes the pulse wave signal into rectangular waves (square waves) to output the shaped pulse wave signal to the CPU 308. The action noise signal shaper 307 shapes the action noise signal into rectangular waves (square waves) to output the shaped action noise signal to the CPU 308.

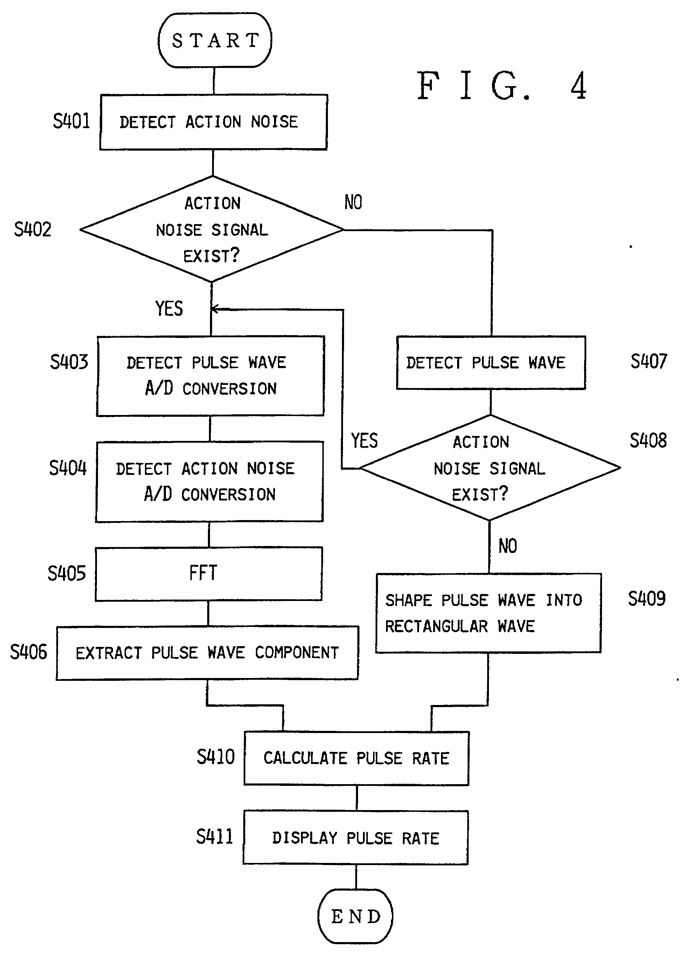

Figure 4 is a flow chart for representing such a sequential operation in which a confirmation is made as to whether or not the action noise signal is present, the calculation method for the pulse wave signal is changed, and the pulse rate is calculated and then is displayed. In Figure 3 and Figure 4, the CPU 308 confirms whether or not the action noise signal is present in response to the output signal issued from the action noise signal shaper 307, and then switches the calculation method (S402). When the existence of the action noise signal is confirmed (S402: YES), the CPU 308 performs an FFT process (S405) to the A/D-converted pulse wave signal (S403) and also A/D-converted action noise signal (S404); and further extracts the pulse wave frequency components (S406).

When the existence of the action noise signal is not confirmed (S402:NO), the pulse wave is detected (S407) and the pulse waveform is shaped (S409). During this process operation, another confirmation is made once or more as to whether or not the action noise signal is present (S408). When there is still no action noise signal (S408:NO), the pulse rate is calculated directly from the rectangular wave (S410). At this time, in Figure 3, since both the pulse wave signal and the action noise signal are no longer required for the AID conversion, the operation of the A/D converter 305 is stopped and also the operation of the multiplier 310 is stopped which is required to perform the FFT process. Furthermore, the process operation required to extract the pulse wave component executed in the CPU 308 is stopped, so that the total power consumption can be lowered.

When there is the action noise signal (S408:YES), the frequency analysis is carried out by the FFT process operation (S405), and the pulse rate is calculated from the extracted pulse wave component (S410).

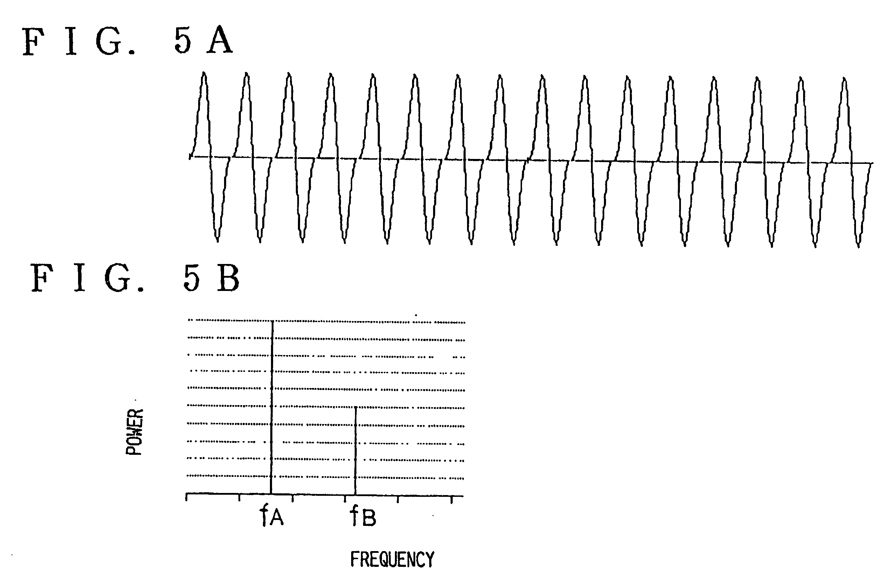

In Figures 5A and 5B, there is shown the result (Figure 5B) of FFT-processing a signal (Figure 5A) to which a frequency component fA and a frequency component fB, equal to a half of the amplitude of the frequency component fA, have been added. The lowest frequency obtained from the FFT-processed result is determined by calculating the inverse of the analyzing period of time. Assuming now that the analyzing period is 16 seconds, the line spectrum resolution becomes 1/16 seconds or 62.5 ms in period. As a consequence, the signal to be analyzed is resolved into harmonic components being equal to 16 Hz multiplied by an integer. The magnitudes (power) of the respective harmonic components are denoted by a height in ordinate. In Figure 5B, it is represented that the power of the frequency component fB is equal to a half of the power of the frequency component fA.

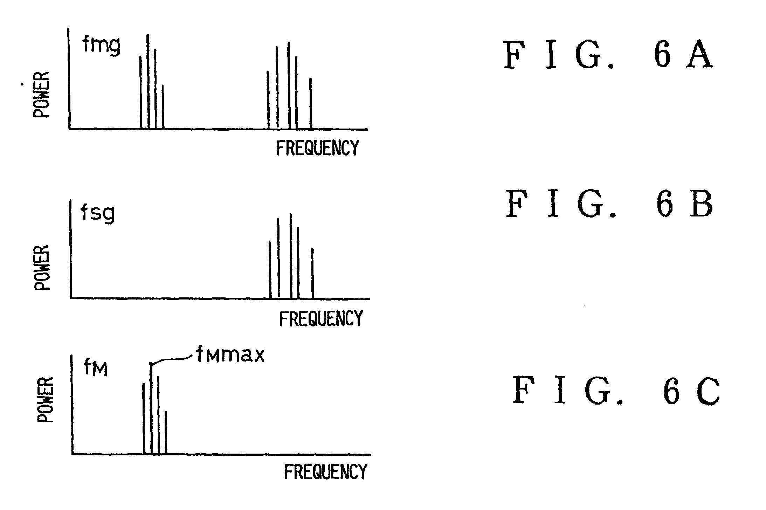

Figures 6A-6C show an example of results obtained by FFT-processing the output signals of the pulse wave sensor and the action noise sensor under action condition. Figure 6A represents a pulse wave spectrum "fmg", Figure 6B indicates an action noise spectrum "fsg", Figure 6C denotes a spectrum obtained by subtracting the action noise spectrum from the pulse wave spectrum, i.e. extracted pulse wave components "fM". In Figure 6A both of the frequency components of the pulse waveform and the frequency components of the signal produced by the action are superimposed. The result shown in Figure 6A is produced by FFT-processing the output signals of the pulse wave sensor. The result shown in Figure 6B is produced by the action noise sensor, and, since the action noise sensor responds only to the action of the biological body, frequency components of the signal produced only by the action are obtained. The spectrum fsg is subtracted from the spectrum fmg, so that among the remaining line spectrum (as shown in Figure 6C), the maximum line spectrum is specified as the pulse wave frequency component. Based upon the frequency component of this pulse wave signal, the pulse rate is calculated (Figure 4, S410) and the calculated pulse rate is output to such a display element as an LCD panel (S411).

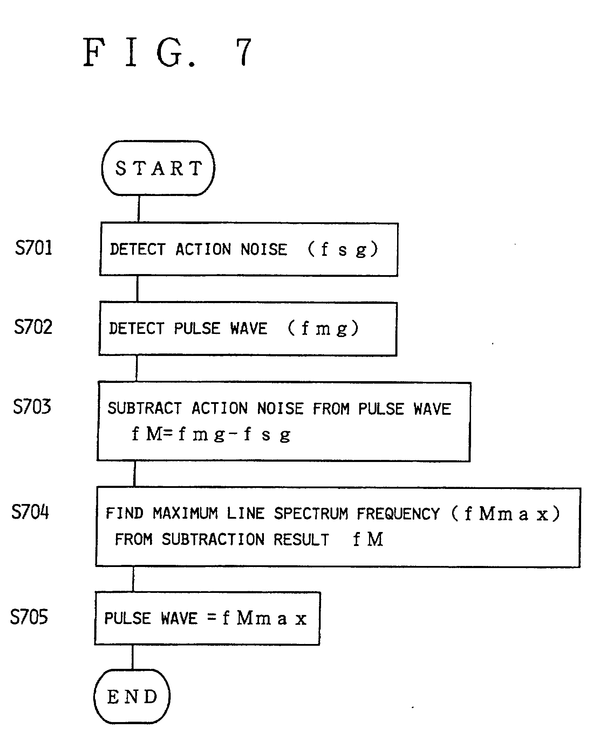

The above-described method for specifying the pulse wave component is shown in Figure 7. At a step S703, the action noise is subtracted from the pulse wave (fM = fmg - fsg), whereby frequency components contained only in the pulse wave signal are derived. The maximum frequency components among the derived pulse wave components fM is specified. The specified fMmax is the frequency component of the pulse wave.

Actually, there are some difficult cases in such a subtracting method for simply subtracting those signal components. When the respective sensor output waveforms are frequency-analyzed, adverse influences are produced by the harmonic signals. As a consequence, a method for specifying a pulse wave will now be described in detail.

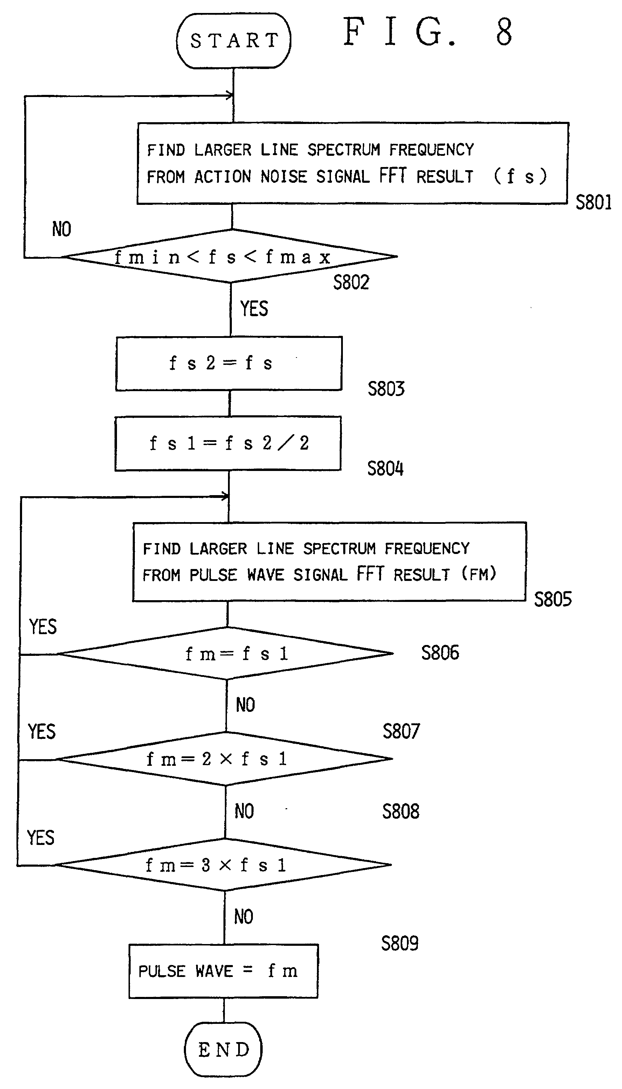

In Figure 8, there is shown such a processing method for specifying a pulse wave component after an action noise component is extracted from a signal of the action noise sensor, and the action noise component is eliminated from a frequency-analyzed result of a pulse wave signal. The second harmonic fs2 of the action noise sensor, which is relatively easy to detect as the action noise component, is specified at steps S801, S802 and S803. Assuming now that the action is "running", fmin of step S802 becomes 2 Hz, corresponding to the lower limit frequency of the second harmonic appearing in the "running". Symbol "fmax" is a frequency determined by the sampling rate for the A/D conversion. Assuming now that the sampling frequency is 8 Hz, the maximum frequency at which the original waveform can be reproduced from the sampling theory is automatically determined as 4 Hz. Within a range from this fmax to fmin, the maximum line spectrum is specified as the second harmonic of the action noise component. As a step S804, the fundamental harmonic fs1 of the action noise component is obtained.

At the subsequent steps S805, S806, S807 and S808, pulse wave components coincident with the fundamental harmonic (fs1), the second harmonic (2 x fs1), and the third harmonic (3 x fs1) of the action noise component are eliminated, and the maximum frequency component among the remaining components is specified as a pulse wave "fm" at a step S809. Normally, the action noise frequency is 1 to 2 Hz. As a result, when the maximum frequency component fmax is equal to 4 Hz, it is sufficient to check the harmonics up to the third harmonic.

Now a description will be made of the reason why the maximum action noise component in the frequency region from 2 Hz to 4 Hz is extracted, and then this frequency is handled as the second harmonic of the action noise component in the above-described embodiment.

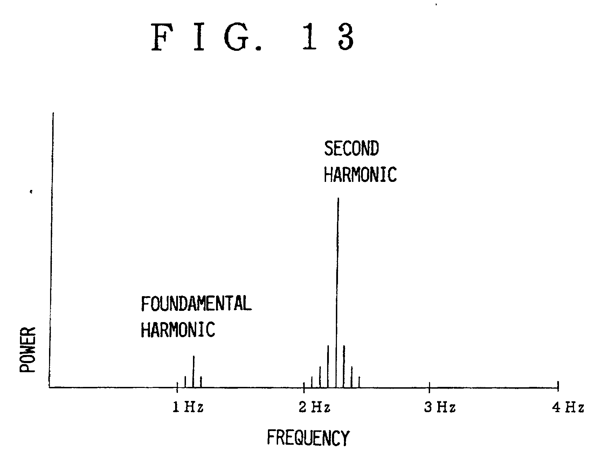

Figure 13 schematically reprsents results obtained by FFT-processing the outputs of the action noise sensor. Generally speaking, under action conditions, especially, under a running condition, as illustrated in Figure 13, a higher power of the second harmonic is obtained, as compared with that of the fundamental harmonic (i.e. 3 to 10 times higher than the fundamental harmonic during normal running). There are three sensing factors of the action noise sensor under running conditions.

That is to say,
(1) up/down movement under running conditions,
(2) fundamental harmonic of arm swings, and
(3) second harmonic leg of arm swings.

As the factor (1), since the up/down movement is equally performed when the right leg is stepped and the left leg is stepped, it becomes the second harmonic of the action noise components.

As to the factor (2), it is directed to such pendulum motion that the arm's pushing motion and the arm's pulling motion constitute one pertiod. However, in practice the arm swings under normal running are not made as a smooth pendulum motion, and therefore power of this component is weakened.

As to the factor (3), since acceleration is instantaneously applied to the arm's pushing motion and the arm's pulling motion, the power of the second harmonic becomes higher than that of the fundamental harmonic. As a consequence, the second harmonic component among the action noise frequency component is emphasized.

Under normal running conditions, when the frequency range is selected from 2 Hz to 4 Hz, the range where the second harmonic appears can be covered, even if the running pace is increased and/or decreased. Therefore, the precision of detection may be increased by extracting the second harmonic component with the featured portion while limited to this frequency range.

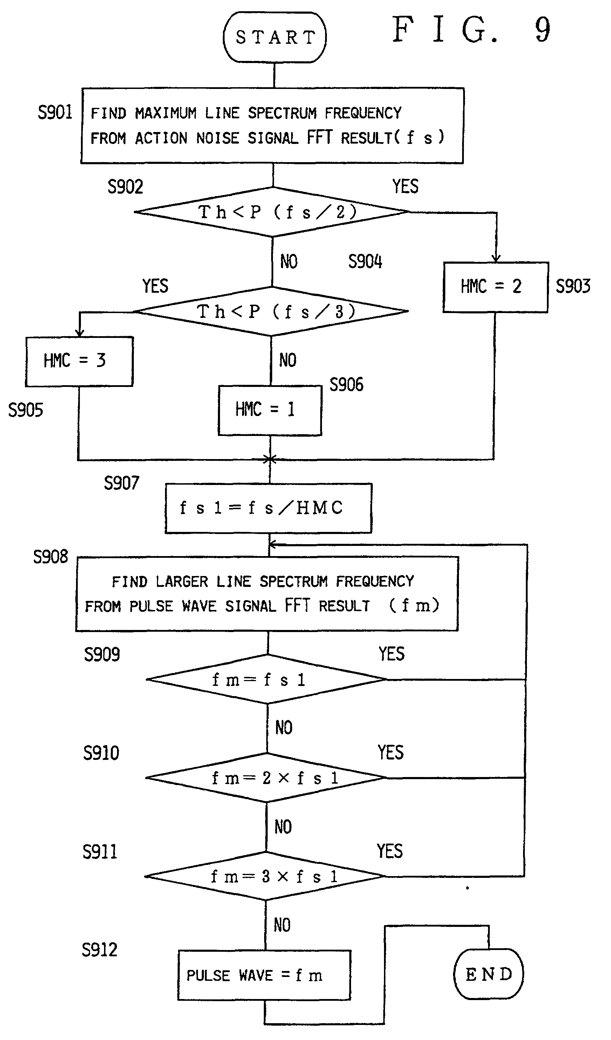

In Figure 9, there is shown a process method for specifying a pulse wave component after the harmonics waves of an action noise signal are specified. At a step S901, a line spectrum fs having a maximum power "P" is obtained from the frequency analayzing result of the action noise signal. Subsequently, a check is made as to whether or not an action noise component P(fs/2) higher than a predetermined value Th exists at a frequency equal to a half of fs (S902). When the line spectrum fs/2 is present (S902:YES), fs is specified as the second harmonic wave (HMC = 2) (S903). When the line spectrum fs/2 is not present (S902:NO), another check is done as to whether or not an action noise component P(fs/3) higher than a predetermined value Th exists at a frequency equal to one third of fs at a step S904. When there is such an action noise component P(fs/3) (S904:YES), fs is specified as the third harmonic wave (HMC = 3) (S905). If there is no action noise component P(fs/3) (S904:NO), then fs is specified as the fundamental harmonic fs1 (S906).

Since it could be specified in the above-described process operations that fs corresponds to one of the higher harmonics, the fundamental harmonic fs1 of the action noise signal is obtained at a step S907. Then, at a step S908, the frequency components fm are successively compared with the action noise frequency in the order of the line spectrum with the higher power P from the frequency-analyzing results of the pulse waves. A check is done as to whether or not this frequency component is coincident with the fundamental harmonic (fs1) of the action noise signal, the second harmonic (2 x fs1), and the third harmonic (3 x fs1) (steps S908, S909, S910 and S911). With this process operation, the maximum pulse wave frequency component fm which is not coincident with the action noise components can be extracted at a step S912.

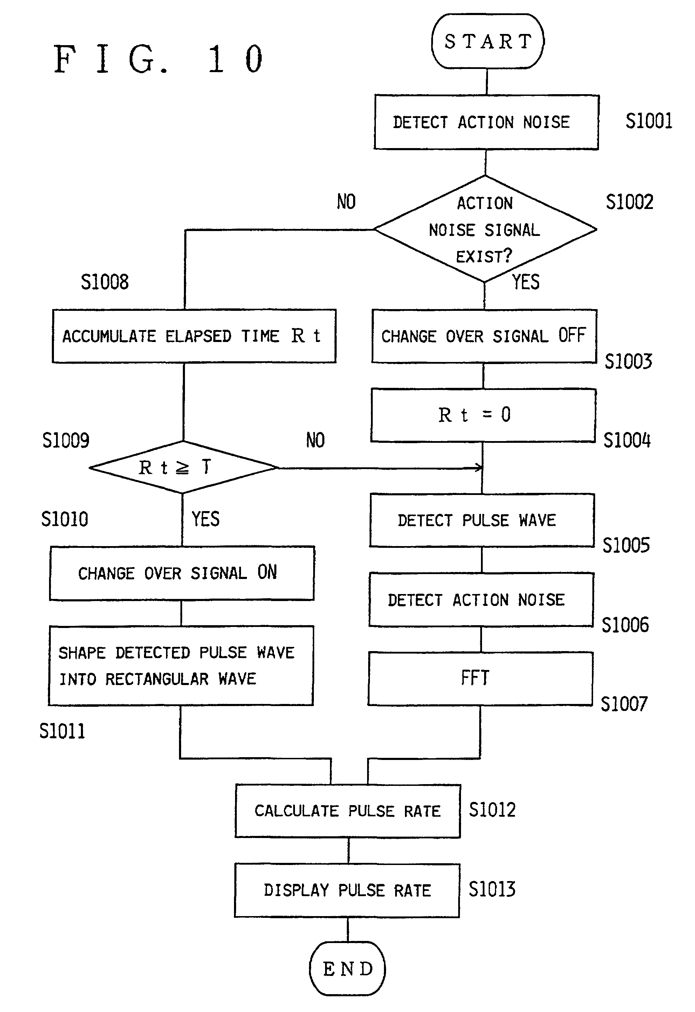

Figure 10 is a flow chart for representing such a sequential operation in which a confirmation is made as to whether or not there is an action noise signal; when such a condition that the action noise signal is not confirmed exceeds a predetermined time "T", the calculation method is changed; and a pulse rate is calculated based on a rectangular-shaped signal of pulse wave, and displayed. In Figure 3 and Figure 10, the CPU 308 makes a confirmation as to whether or not the action noise signal is present based on the output signal derived from the action noise signal shaper 307 (S1002). While the action noise signal is being confirmed (S1002:YES), a change over signal is set to OFF (S1003) so that accumulation time "Rt" for no action noise signal is set to zero (S1004). Thereafter, both the AID converted action noise signal and pulse wave signal are FFT-processed to extract a pulse wave (S1007). Subsequently, a pulse rate is calculated (S1012) to be output to a display device 313 (S1013).

When no action noise signal is confirmed (S1002:NO), the time counting operation is commenced based on the output signal derived from an oscillator 311 and a frequency divider 312, so that elapsed time Rt is accumulated (S1008). When the accumulated time Rt exceeds a certain constant time T which is determined by either the sampling period of the signal used in the FFT process, or the sampling number (S1009:YES), the method for calculating the pulse rate is changed (S1010), the operations of the A/D conversion and the FFT process are stopped, and the pulse wave signal shaped by the pulse wave signal shaper 306 is input to the CPU 308 to calculate the pulse rate (S1012). Then, the calculated pulse rate is output to the display device 313 (S1013).

As described above, there has been explained an embodiment in which the maximum pulse wave frequency component which is not coincident with the action noise component is specified to realize the pulse rate monitor.

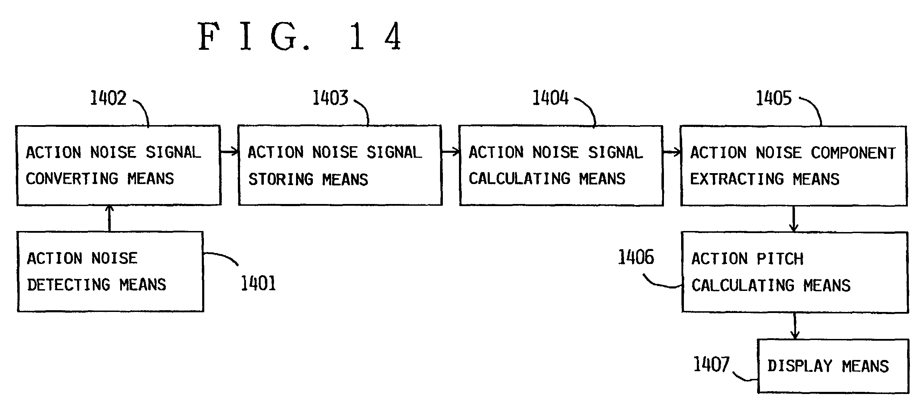

Alternatively, it is possible to construct a high-precision action pitch monitor which employs the same structure as above. In Figure 14, there is shown a functional block diagram of an arrangement of an action pitch monitor. The frequency component of the action pitch is specified by action noise extracting means 1405. This specifying method may be readily realized by way of the methods previously described with reference to Figure 8 and Figure 9. In accordance with the action pitch monitor, running pitches, for example, corresponding to useful information for a runner can be correctly obtained. Also, it may be possible to calculate a running distance based on the running pitches and the length of steps.

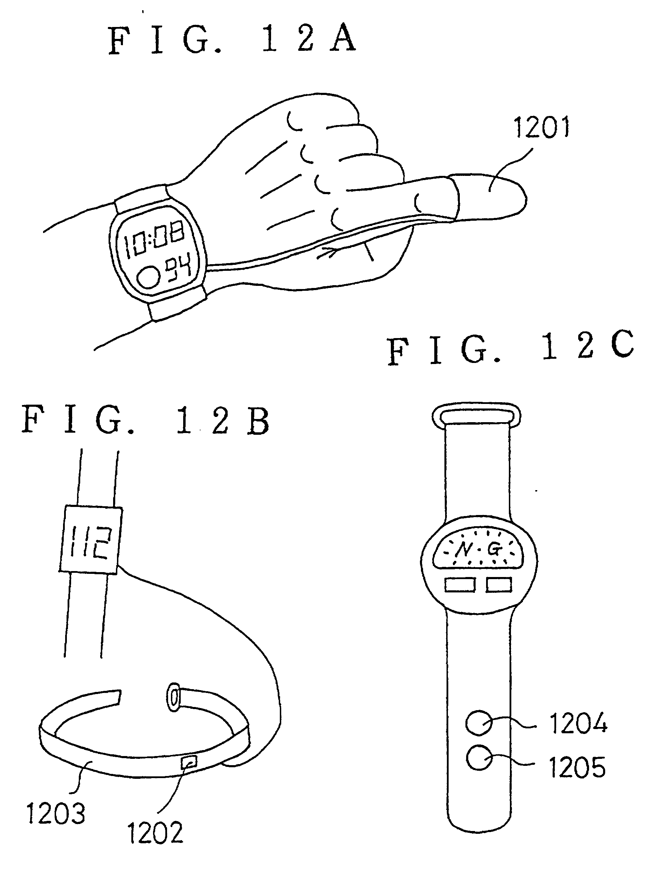

Figures 12A-12C are explanatory diagrams for indicating an example of the outer appearance of a pulse rate monitor or action pitch monitor. Figure 12A indicates a type of pulse rate monitor in which the pulse wave sensor is provided in a finger sack. Figure 12B shows a type of pulse rate monitor in which an action noise sensor 1202 is provided on a belt 1203. Figure 12C indicates a type of pulse wave monitor in which both a pulse wave sensor 1204 and an action noise sensor 1205 are assembled into a wrist watch band in an integral form.

As described above, a pulse wave component extracting means is employed which extracts the pulse wave components from the respective frequency-analyzed results of the pulse wave sensor and the action noise sensor, so that the pulse rate during motion condition can be displayed. Furthermore, under a no motion condition, where the frequency analyzing process operation is not required, the operations of the AID converter and the calculating circuit (which have a relatively high power consumption) can be stopped. Accordingly, the power consumption can be reduced. Also, since a constant time required to perform the frequency analysis is prepared from the stationary condition until the calculation method is switched, so that non-contradictory process operation may be carried out and thus a correct pulse rate can be displayed.

## Claims

1. A pulse rate monitor comprising:
a pulse wave sensor (101, 201, 301) for detecting a pulse from a biological body and generating a signal indicative thereof;
a movement sensor (102, 202, 302) for sensing movement of the biological body and generating a signal indicative thereof;
frequency analysing means (104, 203-8, 308) for resolving the pulse wave indicative signal and movement indicative signal into frequency components (fmg, fsg); and
pulse wave component extracting means for operatively identifying the pulse rate of the biological body from the pulse wave frequency components and movement frequency components; **characterised in that**:
said movement sensor comprises an action noise sensor for detecting an action noise corresponding to a biological body action, and for outputting a movement signal derived from said action noise; and
said pulse wave component extracting means is adapted to determine the pulse rate in accordance with a said biological body action by extracting the typical pulse wave component from the pulse wave components and the movement signal components and further comprises pulse rate calculating means for calculating pulse rate per predetermined period of the biological body based on the typical pulse wave component extracted by said pulse wave component extracting means.

2. A pulse rate monitor as claimed in claim 1 wherein said pulse wave sensor senses variation in blood pressure of said body.

3. A pulse rate monitor as claimed in claim 1 or claim 2, wherein said pulse wave component extracting means (104,209,308) comprises:
subtracting means for calculating a subtraction between the pulse wave frequency components and the movement frequency components; and
pulse wave maximum component extracting means for extracting the typical pulse rate of the body having a maximum power spectrum from pulse wave components obtained by said subtracting means.

4. A pulse rate monitor as claimed in any preceding claim wherein said frequency analyzing means (104,203-8,308) comprises:
pulse wave signal converting means (203) for converting the pulse wave indicative signal into a digital signal;
movement indicative signal converting means (204) for converting the movement indicative signal into a digital signal;
pulse wave signal storing means (205) for storing the digital signal converted by said pulse wave signal converting means (203);
movement indicative signal storing means (206) for storing the digital signal converted by said movement indicative signal converting means (204);
pulse wave signal calculating means (207) for analyzing the digital signal stored in said pulse wave signal storing means (205) to obtain the pulse wave components representing frequency and power spectrum of the pulse wave signal; and
movement indicative signal calculating means (208) for analyzing the digital signal stored in said movement indicative signal storing means (206) to obtain the movement indicative components representing frequency and power spectrum of the movement indicative signal;
and wherein said pulse wave component extracting means (104,209,308) receives the pulse wave components by said pulse wave signal calculating means (207) and the movement indicative components by said movement indicative signal calculating means (208).

5. A pulse rate monitor as claimed in claim 4, wherein said pulse wave component extracting means (104,209,308) comprises:
movement indicative components eliminating means for eliminating the movement indicative components from the pulse wave components output from said pulse wave signal calculating means (207); and
pulse wave maximum component extracting means for extracting the typical pulse wave component having a maximum power spectrum from pulse wave components obtained by said movement indicative components eliminating means.

6. A pulse rate monitor as claimed in claim 4 or 5, wherein said pulse wave component extracting means (104,209,308) comprises:
movement indicative maximum component extracting means for extracting a typical movement indicative component having a maximum power spectrum from the movement indicative noise components obtained by said movement indicative signal calculating means (208);
harmonic specifying means for discriminating to which order of harmonic the typical movement indicative component obtained by said movement indicative maximum component extracting means corresponds, including the fundamental harmonic, and for specifying harmonic components including fundamental harmonic component of the movement indicative components;
movement indicative component eliminating means for eliminating all of harmonic components specified by said harmonic specifying means from the pulse wave components obtained by said pulse wave signal calculating means (207); and
pulse wave maximum component extracting means for extracting a typical pulse wave component having a maximum power spectrum from the pulse wave components obtained by said movement indicative component eliminating means.

7. A pulse rate monitor as claimed in claims 4, 5 or 6, wherein said pulse wave component extracting means (104,209,308) comprises:
movement indicative maximum component extracting means for extracting a typical movement indicative component having a maximum power spectrum from the movement indicative components obtained by said movement indicative signal calculating means (208);
harmonic specifying means for identifying the typical movement indicative component obtained by said movement indicative maximum component extracting means as a second order harmonic, and for specifying harmonic components including the fundamental harmonic components of the movement indicative components;
movement indicative component eliminating means for eliminating all of harmonic components specified by said harmonic specifying means from the pulse wave components obtained by said pulse wave signal calculating means (207); and
pulse wave maximum component extracting means for extracting a typical pulse wave component having a maximum power spectrum from the pulse wave components obtained by said movement indicative component eliminating means.

8. A pulse rate monitor as claimed in claims 4, 5, 6 or 7, wherein said pulse wave signal calculating means (207) and said movement indicative signal calculating means (208) both perform Fast Fourier Transform operations.

9. A pulse rate monitor as claimed in any preceding claim, further comprising:
timing means (211) for counting and accumulating elapsed time under a condition of no movement indicative signal being detected, and for outputting a change over signal when the elapsed time exceeds a predetermined time period; and
calculation changing means (210) for changing an input signal to said processing means from the typical pulse wave component to the pulse wave signal output by said pulse wave sensor in response to the change over signal obtained by said timing means (211).

10. An action pitch monitor comprising:
action noise detecting means (1401) having an action noise sensor for detecting an action noise corresponding to a biological body action, and for outputting an action noise signal;
action noise signal converting means (1402) for converting the action noise signal into a digital signal;
action noise signal storing means (1403) for storing the digital signal converted by said action noise signal converting means (1402);
action noise signal calculating means (1404) for processing the digital signal stored in said action noise signal storing means (1403) to obtain the action noise components representing frequency and power spectrum of the action noise signal;
action noise component extracting means (1405) for extracting a typical action noise component from the action noise components; and
action pitch calculating means (1406) for calculating action pitch, per predetermined period, of the biological body based on the typical action noise components extracted by said action noise component extracting means (1405).

## Patentansprüche

1. Pulsratenmonitor, umfassend:
einen Pulswellensensor (101, 201, 301) zum Erfassen eines Pulses von einem biologischen Körper und zum Erzeugen eines Signals, das diesen anzeigt;
einen Bewegungssensor (102, 202, 302) zum Erfassen der Bewegung des biologischen Körpers und zum Erzeugen eines Signals, das diese anzeigt;
ein Frequenzanalysemittel (104, 203-8, 308) zum Auflösen des die Pulswelle anzeigenden Signals und des die Bewegung anzeigenden Signals in Frequenzkomponenten (fmg, fsg); und
ein Pulswellenkomponenten-Extraktionsmittel zum operativen Identifizieren der Pulsrate des biologischen Körpers anhand der Pulswellenfrequenzkomponenten und der Bewegungsfrequenzkomponenten; **dadurch gekennzeichnet, daß**:
der Bewegungssensor einen Aktionsgeräuschsensor umfaßt zum Erfassen eines Aktionsgeräusches, das einer Aktion des biologischen Körpers zugeordnet ist, und zum Ausgeben eines Bewegungssignals, das aus dem Aktionsgeräusch abgeleitet wird; und
das Pulswellenkomponenten-Extraktionsmittel dafür konfiguriert ist, die Pulsrate entsprechend einer Aktion des biologischen Körpers zu ermitteln durch Extrahieren der typischen Pulswellenkomponente aus den Pulswellenkomponenten und den Bewegungssignalkomponenten, und ferner ein Pulsratenberechnungsmittel umfaßt zum Berechnen der Pulsrate pro vorgegebener Periode des biologischen Körpers auf der Grundlage der vom Pulswellenkomponenten-Extraktionsmittel extrahierten typischen Pulswellenkomponente.

2. Pulsratenmonitor nach Anspruch 1, bei dem der Pulswellensensor eine Änderung des Blutdrucks des Körpers erfaßt.

3. Pulsratenmonitor nach Anspruch 1 oder Anspruch 2, bei dem das Pulswellenkomponenten-Extraktionsmittel (104, 209, 308) umfaßt:
ein Subtraktionsmittel zum Berechnen einer Subtraktion zwischen den Pulswellenfrequenzkomponenten und den Bewegungsfrequenzkomponenten; und
ein Pulswellen-Maximalkomponenten-Extraktionsmittel zum Extrahieren der typischen Pulsrate des Körpers mit einem maximalen Leistungsspektrum aus den vom Subtraktionsmittel erhaltenen Pulswellenkomponenten.

4. Pulsratenmonitor nach irgendeinem der vorangehenden Ansprüche, bei dem das Frequenzanalysemittel (104, 203-8, 308) umfaßt:
ein Pulswellensignal-Umsetzungsmittel (203) zum Umsetzen des die Pulswelle anzeigenden Signals in ein digitales Signal;
ein Bewegungsanzeigesignal-Umsetzungsmittel (204) zum Umsetzen des die Bewegung anzeigenden Signals in ein digitales Signal;
ein Pulswellensignal-Speichermittel (205) zum Speichern des vom Pulswellensignal-Umsetzungsmittel (203) umgesetzten digitalen Signals;
ein Bewegungsanzeigesignal-Speichermittel (206) zum Speichern des vom Bewegungsanzeigesignal-Umsetzungsmittel (204) umgesetzten digitalen Signals;
ein Pulswellensignal-Berechnungsmittel (207) zum Analysieren des im Pulswellensignal-Speichermittel (205) gespeicherten digitalen Signals, um die Pulswellenkomponenten zu erhalten, die die Frequenz und das Leistungsspektrum des Pulswellensignals repräsentieren; und
ein Bewegungsanzeigesignal-Berechnungsmittel (208) zum Analysieren des im Bewegungsanzeigesignal-Speichermittel (206) gespeicherten digitalen Signals, um die die Bewegung anzeigenden Komponenten zu erhalten, die die Frequenz und das Leistungsspektrum des die Bewegung anzeigenden Signals repräsentieren;
wobei das Pulswellenkomponenten-Extraktionsmittel (104, 209, 308) die Pulswellenkomponenten mittels des Pulswellensignal-Berechnungsmittels (207) und die die Bewegung anzeigenden Komponenten mittels des Bewegungsanzeigesignal-Berechnungsmittels (208) empfängt.

5. Pulsratenmonitor nach Anspruch 4, bei dem das Pulswellenkomponenten-Extraktionsmittel (104, 209, 308) umfaßt:
ein Bewegungsanzeigekomponenten-Eliminierungsmittel zum Eliminieren der die Bewegung anzeigenden Komponenten aus den Pulswellenkomponenten, die vom Pulswellensignal-Berechnungsmittel (207) ausgegeben werden; und
ein Pulswellen-Maximalkomponenten-Extraktionsmittel zum Extrahieren der typischen Pulswellenkomponente mit einem maximalen Leistungsspektrum aus den Pulswellenkomponenten, die vom Bewegungsanzeigekomponenten-Eliminierungsmittel erhalten werden.

6. Pulsratenmonitor nach Anspruch 4 oder 5, bei dem das Pulswellenkomponenten-Extraktionsmittel (104, 209, 308) umfaßt:
ein Bewegungsanzeige-Maximalkomponenten-Extraktionsmittel zum Extrahieren einer typischen Bewegungsanzeigekomponente mit einem maximalen Leistungsspektrum aus den Bewegungsanzeige-Geräuschkomponenten, die vom Bewegungsanzeigesignal-Berechnungsmittel (208) erhalten werden;
ein Oberwellenspezifizierungsmittel zum Unterscheiden, welcher Oberwellenordnung die typische Bewegungsanzeigekomponente entspricht, die vom Bewegungsanzeige-Maximalkomponenten-Extraktionsmittel erhalten worden ist, einschließlich der Grundwelle, und zum Spezifizieren der Oberwellenkomponenten einschließlich der Grundwellenkomponente der Bewegungsanzeigekomponenten,
ein Bewegungsanzeigekomponenten-Eliminierungsmittel zum Eliminieren aller Oberwellenkomponenten, die vom Oberwellenspezifizierungsmittel spezifiziert worden sind, aus den Pulswellenkomponenten, die vom Pulswellensignal-Berechnungsmittel (207) erhalten worden sind; und
ein Pulswellen-Maximalkomponenten-Extraktionsmittel zum Extrahieren einer typischen Pulswellenkomponente mit einem maximalen Leistungsspektrum aus den Pulswellenkomponenten, die vom Bewegungsanzeigekomponenten-Eliminierungsmittel erhalten worden sind.

7. Pulsratenmonitor nach einem der Ansprüche 4,5 oder 6, bei dem das Pulswellenkomponenten-Extraktionsmittel (104, 209, 308) umfaßt:
ein Bewegungsanzeige-Maximalkomponenten-Extraktionsmittel zum Extrahieren einer typischen Bewegungsanzeigekomponente mit einem maximalen Leistungsspektrum aus den Bewegungsanzeigekomponenten, die vom Bewegungsanzeigesignal-Berechnungsmittel (208) erhalten werden;
ein Oberwellenspezifierungsmittel zum Identifizieren der typischen Bewegungsanzeigekomponente, die vom Bewegungsanzeige-Maximalkomponenten-Extraktionsmittel als eine Oberwelle zweiter Ordnung erhalten worden ist, und zum Spezifizieren der Oberwellenkomponenten einschließlich der Grundwellenkomponenten der Bewegungsanzeigekomponenten;
ein Bewegungsanzeigekomponenten-Eliminierungsmittel zum Eliminieren aller Oberwellenkomponenten, die vom Oberwellenspezifizierungsmittel spezifiziert werden, aus den Pulswellenkomponenten, die vom Pulswellensignal-Berechnungsmittel (207) erhalten werden; und
ein Pulswellen-Maximalkomponenten-Extraktionsmittel zum Extrahieren einer typischen Pulswellenkomponente mit einem maximalen Leistungsspektrum aus den vom Bewegungsanzeigekomponenten-Eliminierungsmittel erhaltenen Pulswellenkomponenten.

8. Pulsratenmonitor nach einem der Ansprüche 4, 5, 6 oder 7, bei dem das Pulswellensignal-Berechnungsmittel (207) und das Bewegungsanzeigesignal-Berechnungsmittel (208) beide schnelle Fourier-Transformationsoperationen durchführen.

9. Pulsratenmonitor nach irgendeinem der vorangehenden Ansprüche, der femer umfaßt:
ein Zeitsteuerungsmittel (211) zum Zählen und Akkumulieren der verstrichenen Zeit unter einer Bedingung, in der kein Bewegungsanzeigesignal erfaßt wird, und zum Ausgeben eines Wechselsignals, wenn die verstrichene Zeitspanne eine vorgegebene Zeitperiode überschreitet; und
ein Berechnungsänderungsmittel (210) zum Ändern eines Eingangssignals für das Verarbeitungsmittel von der typischen Pulswellenkomponente zum Pulswellensignal, das vom Pulswellensensor ausgegeben wird, in Reaktion auf das vom Zeitsteuerungsmittel (211) erhaltene Wechselsignal.

10. Aktionsschrittzahlmonitor, umfassend:
ein Aktionsgeräuscherfassungsmittel (1401) mit einem Aktionsgeräuschsensor zum Erfassen eines Aktionsgeräusches, das einer Aktion eines biologischen Körpers zugeordnet ist, und zum Ausgeben eines Aktionsgeräuschsignals;
ein Aktionsgeräuschsignal-Umsetzungsmittel (1402) zum Umsetzen des Aktionsgeräuschsignals in ein digitales Signal;
ein Aktionsgeräuschsignal-Speichermittel (1403) zum Speichern des vom Aktionsgeräuschsignal-Umsetzungsmittel (1402) umgesetzten digitalen Signals;
ein Aktionsgeräuschsignal-Berechnungsmittel (1404) zum Verarbeiten des im Aktionsgeräuschsignal-Speichermittel (1403) gespeicherten digitalen Signals, um die Aktionsgeräuschkomponenten zu erhalten, die die Frequenz und das Leistungsspektrum des Aktionsgeräuschsignals repräsentieren;
ein Aktionsgeräuschkomponenten-Extraktionsmittel (1405) zum Extrahieren einer typischen Aktionsgeräuschkomponente aus den Aktionsgeräuschkomponenten; und
ein Aktionsschrittzahl-Berechnungsmittel (1406) zum Berechnen der Aktionsschrittzahl pro vorgegebener Periode des biologischen Körpers auf der Grundlage der vom Aktionsgeräuschkomponenten-Extraktionsmittel (1405) extrahierten typischen Aktionsgeräuschkomponenten.

## Revendications

1. Dispositif de contrôle de rythme de pouls, comprenant :
un capteur d'onde de pouls (101, 201, 301) pour détecter le pouls en provenance d'un corps biologique et pour produire un signal indicatif de ce dernier ;
un capteur de mouvement (102, 202, 302) pour détecter un mouvement du corps biologique et pour produire un signal indicatif de ce dernier ;
des moyens d'analyse de fréquence (104, 203-8, 308) pour séparer le signal indicatif d'onde de pouls et le signal indicatif de mouvement en composantes de fréquence (fmg, fsg) ; et
des moyens d'extraction de composante d'onde de pouls pour identifier de manière opérationnelle le rythme de pouls du corps biologique à partir des composantes de fréquence d'onde de pouls et des composantes de fréquence de mouvement ; **caractérisé en ce que** :
ledit capteur de mouvement comprend un capteur de bruit d'action pour détecter un bruit d'action correspondant à une action de corps biologique, et pour sortir un signal de mouvement obtenu à partir dudit bruit d'action ; et
lesdits moyens d'extraction de composante d'onde de pouls sont conçus pour déterminer le rythme de pouls selon une dite action de corps biologique en extrayant la composante typique d'onde de pouls à partir des composantes d'onde de pouls et des composantes de signal de mouvement, et comprennent de plus des moyens de calcul de rythme de pouls pour calculer un rythme de pouls par période prédéterminée du corps biologique sur la base de la composante typique d'onde de pouls extraite par lesdits moyens d'extraction de composante d'onde de pouls.

2. Dispositif de contrôle de rythme de pouls selon la revendication 1, dans lequel ledit capteur d'onde de pouls détecte une variation de pression sanguine dudit corps.

3. Dispositif de contrôle de rythme de pouls selon la revendication 1 ou la revendication 2, dans lequel lesdits moyens d'extraction de composante d'onde de pouls (104, 209, 308) comprennent :
des moyens de soustraction pour calculer une soustraction entre les composantes de fréquence d'onde de pouls et les composantes de fréquence de mouvement ; et
des moyens d'extraction de composante maximale d'onde de pouls pour extraire le rythme typique de pouls du corps ayant un spectre d'énergie maximale à partir de composantes d'onde de pouls obtenues par lesdits moyens de soustraction.

4. Dispositif de contrôle de rythme de pouls selon l'une quelconque des revendications précédentes, dans lequel lesdits moyens d'analyse de fréquence (104, 203-8, 308) comprennent :
des moyens de transformation de signal d'onde de pouls (203) pour transformer le signal indicatif d'onde de pouls en un signal numérique ;
des moyens de transformation de signal indicatif de mouvement (204) pour transformer le signal indicatif de mouvement en un signal numérique ;
des moyens de stockage de signal d'onde de pouls (205) pour stocker le signal numérique transformé par lesdits moyens de transformation de signal d'onde de pouls (203) ;
des moyens de stockage de signal indicatif de mouvement (206) pour stocker le signal numérique transformé par lesdits moyens de transformation de signal indicatif de mouvement (204) ;
des moyens de calcul de signal d'onde de pouls (207) pour analyser le signal numérique stocké dans lesdits moyens de stockage de signal d'onde de pouls (205) pour obtenir les composantes d'onde de pouls représentant la fréquence et le spectre d'énergie du signal d'onde de pouls ; et
des moyens de calcul de signal indicatif dé mouvement (208) pour analyser le signal numérique stocké dans lesdits moyens de stockage de signal indicatif de mouvement (206) pour obtenir les composantes indicatives de mouvement représentant la fréquence et le spectre d'énergie du signal indicatif de mouvement ;
et dans lequel lesdits moyens d'extraction de composante d'onde de pouls (104, 209, 308) reçoivent les composantes d'onde de pouls par lesdits moyens de calcul de signal d'onde de pouls (207) et les composantes indicatives de mouvement par lesdits moyens de calcul de signal indicatif de mouvement (208).

5. Dispositif de contrôle de rythme de pouls selon la revendication 4, dans lequel lesdits moyens d'extraction de composante d'onde de pouls (104, 209, 308) comprennent :
des moyens d'élimination de composantes indicatives de mouvement pour éliminer les composantes indicatives de mouvement des composantes d'onde de pouls sorties en provenance desdits moyens de calcul de signal d'onde de pouls (207) ; et
des moyens d'extraction de composante maximale d'onde de pouls pour extraire la composante typique d'onde de pouls ayant un spectre d'énergie maximale à partir de composantes d'onde de pouls obtenues par lesdits moyens d'élimination de composantes indicatives de mouvement.

6. Dispositif de contrôle de rythme de pouls selon la revendication 4 ou 5, dans lequel lesdits moyens d'extraction de composante d'onde de pouls (104, 209, 308) comprennent :
des moyens d'extraction de composante maximale indicative de mouvement pour extraire une composante typique indicative de mouvement ayant un spectre d'énergie maximale à partir des composantes de bruit indicatives de mouvement obtenues par lesdits moyens de calcul de signal indicatif de mouvement (208) ;
des moyens de spécification d'harmonique pour discriminer à quel ordre d'harmonique la composante typique indicative de mouvement obtenue par lesdits moyens d'extraction de composante maximale indicative de mouvement correspond, comprenant l'harmonique fondamentale, et pour spécifier des composantes harmoniques comprenant la composante d'harmonique fondamentale des composantes indicatives de mouvement ;
des moyens d'élimination de composante indicative de mouvement pour éliminer toutes les composantes harmoniques spécifiées par lesdits moyens de spécification d'harmonique à partir des composantes d'onde de pouls obtenues par lesdits moyens de calcul de signal d'onde de pouls (207) ; et
des moyens d'extraction de composante maximale d'onde de pouls pour extraire une composante typique d'onde de pouls ayant un spectre d'énergie maximale à partir des composantes d'onde de pouls obtenues par lesdits moyens d'élimination de composante indicative de mouvement.

7. Dispositif de contrôle de rythme de pouls selon les revendications 4, 5 ou 6, dans lequel lesdits moyens d'extraction d'onde de pouls (104, 209, 308) comprennent :
des moyens d'extraction de composante maximale indicative de mouvement pour extraire une composante typique indicative de mouvement ayant un spectre d'énergie maximale à partir des composantes indicatives de mouvement obtenues par lesdits moyens de calcul de signal indicatif de mouvement (208) ;
des moyens de spécification d'harmonique pour identifier la composante typique indicative de mouvement obtenue par lesdits moyens d'extraction de composante maximale indicative de mouvement en tant qu'harmonique de deuxième ordre, et pour spécifier des composantes harmoniques comprenant les composantes d'harmonique fondamentale des composantes indicatives de mouvement ;
des moyens d'élimination de composante indicative de mouvement pour éliminer toutes les composantes harmoniques spécifiées par lesdits moyens de spécification d'harmonique à partir des composantes d'onde de pouls obtenues par lesdits moyens de calcul de signal d'onde de pouls (207) ; et
des moyens d'extraction de composante maximale d'onde de pouls pour extraire une composante typique d'onde de pouls ayant un spectre d'énergie maximale à partir des composantes d'onde de pouls obtenues par lesdits moyens d'élimination de composante indicative de mouvement.

8. Dispositif de contrôle de rythme de pouls selon les revendications 4, 5, 6 ou 7, dans lequel lesdits moyens de calcul de signal d'onde de pouls (207) et lesdits moyens de calcul de signal indicatif de mouvement (208) effectuent tous les deux des opérations de Transformée de Fourier Rapide.

9. Dispositif de contrôle de rythme de pouls selon l'une quelconque des revendications précédentes, comprenant de plus :
des moyens de chronométrage (211) pour compter et accumuler le temps écoulé sous une condition d'absence de détection de signal indicatif de mouvement, et pour sortir un signal de changement quand le temps écoulé dépasse une période de temps prédéterminée ; et
des moyens de changement de calcul (210) pour changer un signal d'entrée vers lesdits moyens de traitement depuis la composante typique d'onde de pouls vers le signal d'onde de pouls sorti par ledit capteur d'onde de pouls en réponse au signal de changement obtenu par lesdits moyens de chronométrage (211).

10. Dispositif de contrôle de pas d'action, comprenant :
des moyens de détection de bruit d'action (1401) ayant un capteur de bruit d'action pour détecter un bruit d'action correspondant à une action de corps biologique, et pour sortir un signal de bruit d'action ;
des moyens de transformation de signal de bruit d'action (1402) pour transformer le signal de bruit d'action en un signal numérique ;
des moyens de stockage de signal de bruit d'action (1403) pour stocker le signal numérique transformé par lesdits moyens de transformation de signal de bruit d'action (1402) ;
des moyens de calcul de signal de bruit d'action (1404) pour traiter le signal numérique stocké dans lesdits moyens de stockage de signal de bruit d'action (1403) pour obtenir les composantes de bruit d'action représentant la fréquence et le spectre d'énergie du signal de bruit d'action ;
des moyens d'extraction de composante de bruit d'action (1405) pour extraire une composante typique de bruit d'action à partir des composantes de bruit d'action ; et
des moyens de calcul de pas d'action (1406) pour calculer un pas d'action, par période prédéterminée, du corps biologique sur la base des composantes typiques de bruit d'action extraites par lesdits moyens d'extraction de composante de bruit d'action (1405).
